# EUROPEAN PATENT APPLICATION

(11) **EP 4 169 611 A1**
(43) Date of publication of application: **26.04.2023**
(21) Application number: 21826771.4
(22) Date of filing: 08.04.2021
(51) Int. Cl.: B01J 23/652, B22F 1/00, B22F 9/24, B22F 9/30, B82Y 30/00, C01B 3/04, C01B 3/16, C07B 61/00, C07C 1/04, C07C 1/12, C07C 9/04, C22C 5/04, C22C 27/04, C25B 11/054, C25B 11/081, C25B 11/089, H01M 4/90, H01M 8/0606

(54) **PLATINUM-TUNGSTEN SOLID SOLUTION PARTICLES AND CATALYST CONTAINING SAME**

(30) Priority: 19.06.2020 JP 2020105895; 15.09.2020 JP 2020155008
(71) Applicant: Nippon Soda Co., Ltd., Tokyo 100-8165 (JP)
(72) Inventor: KITAGAWA, Hiroshi, Kyoto-shi, Kyoto 606-8501 (JP); KOBAYASHI, Hirokazu, Kyoto-shi, Kyoto 606-8501 (JP); KUSADA, Kohei, Kyoto-shi, Kyoto 606-8501 (JP); WU, Dongshuang, Kyoto-shi, Kyoto 606-8501 (JP); KOBAYASHI, Daiya, Ichihara-shi, Chiba 290-0045 (JP)
(74) Representative: Sonnenhauser, Thomas Martin
(86) International application number: PCT/JP2021/014927
(87) International publication number: WO 2021/256061

(57) **Abstract**

An object of the present invention is to provide platinum-tungsten solid solution particles that can be suitably used for catalyst applications and others. Another object is to provide a catalyst with higher catalytic activity than when platinum is used alone. Disclosed are platinum-tungsten solid solution particles comprising platinum and tungsten in solid solution at an atomic level. Also disclosed is a catalyst comprising the platinum-tungsten solid solution particles.

## Description

### Technical Field

The present invention relates to platinum-tungsten solid solution particles comprising platinum and tungsten in solid solution at an atomic level, and a catalyst containing the platinum-tungsten solid solution particles. The present invention also relates to a hydrogen evolution catalyst, a carbon monoxide hydrogenation catalyst, or a carbon dioxide hydrogenation catalyst. The present application claims the priority of Japanese Patent Application No. 2020-105895, filed on June 19, 2020, and Japanese Patent Application No. 2020-155008, filed on September 15, 2020, the contents of which are herein incorporated by reference.

### Background Art

In recent years, the development of highly efficient catalysts for hydrogen evolution reaction (HER) in acidic media has become very important to promote electrochemical energy conversion in fuel cells and to produce hydrogen as an ideal energy carrier through water splitting. Currently, platinum and platinum-based nanomaterials are considered to be the most advanced HER electrocatalysts due to their high catalytic activity and stability. Various alloy nanoparticles have since been developed, including platinum-ruthenium-nickel solid solution (PtRuNi) (see Non-Patent Document 1), platinum-silver solid solution (PtAg) (see Non-Patent Document 2), or platinum-gold solid solution (PtAu) (see Non-Patent Document 3) nanoparticles. These nanoparticles have been reported to exhibit higher HER activity than platinum nanoparticles.

Meanwhile, tungsten has a large negative redox potential of tungsten cation (E^{θ} is -1.1 V in the case of W⁺⁶→W⁰) . Accordingly, the reduction of tungsten cation to tungsten metal is very difficult. In addition, the resulting tungsten is readily oxidized after exposure to air. Thus, platinum and tungsten are difficult to form a solid solution. The following compounds have recently been reported, including a compound in which platinum is atomically dispersed in oxygen-deficient tungsten oxide (WO₃₋ₓ) or a platinum-tungsten intermetallic compound, namely a Pt₂W compound. However, any platinum-tungsten solid solution has not been developed.

### Prior Art Documents

### Non-Patent Documents

Non-Patent Document 1: Shi, Y.-C.; Yuan, T.; Feng, J.-J.; Yuan, J.; Wang, A.-J. Rapid Fabrication of Support-Free Trimetallic Pt53Ru39Ni8 Nanosponges with Enhanced Electrocatalytic Activity for Hydrogen Evolution and Hydrazine Oxidation Reactions. J. Colloid Interface Sci. 2017, 505, 14-22.
Non-Patent Document 2: Weng, X.; Liu, Q.; Wang, A.-J.; Yuan, J.; Feng, J.-J. Simple One-Pot Synthesis of Solid-Core@porous-Shell Alloyed PtAg Nanocrystals for the Superior Catalytic Activity toward Hydrogen Evolution and Glycerol Oxidation. J. Colloid Interface Sci. 2017, 494, 15-21.
Non-Patent Document 3: Weng, X.; Liu, Y.; Wang, K.-K.; Feng, J.-J.; Yuan, J.; Wang, A.-J.; Xu, Q.-Q. Single-Step Aqueous Synthesis of AuPt Alloy Nanodendrites with Superior Electrocatalytic Activity for Oxygen Reduction and Hydrogen Evolution Reaction. Int. J. Hydrog. Energy 2016, 41 (40), 18193-18202.

### Summary of the Invention

### Object to be Solved by the Invention

An object of the present invention is to provide platinum-tungsten solid solution particles that can be suitably used for catalyst applications and others. Another object is to provide a catalyst with higher catalytic activity than when platinum is used alone.

### Means to Solve the Object

The present inventors have hypothesized that if platinum and tungsten are made to form a solid solution at an atomic level, the catalytic activity for various reactions may be increased when compared to the case of using platinum alone. Then, further research has revealed that pyrolysis-mediated liquid phase synthesis is used to be able to form a solid solution comprising platinum and tungsten in solid solution at an atomic level. Moreover, the above-described solid solution may be obtained as nano-sized particles, and the resulting particles have been found to be entirely composed of a uniform solid solution. The solid solution particles had much higher catalytic activity than in the case of platinum alone. Previously, neither solid solution nanoparticles comprising platinum and tungsten in solid solution at an atomic level nor a catalyst containing solid solution nanoparticles comprising platinum and tungsten in solid solution at an atomic level has been known. The nanoparticles refer to particles with a particle diameter of 1 to 100 nm.

Specifically, the present invention is set forth in the following items.
(1) Platinum-tungsten solid solution particles comprising platinum and tungsten in solid solution at an atomic level.
(2) A catalyst comprising the platinum-tungsten solid solution particles according to the above item (1).
(3) The catalyst according to the above item (2), which is a hydrogen evolution catalyst.
(4) The catalyst according to the above item (2), which is a carbon monoxide hydrogenation catalyst.
(5) The catalyst according to the above item (2), which is a carbon dioxide hydrogenation catalyst.

### Effect of the Invention

The platinum-tungsten solid solution particles of the present invention have excellent catalytic activity. A hydrogen evolution catalyst, a carbon monoxide hydrogenation catalyst, and a carbon dioxide hydrogenation catalyst of the present invention each contain the solid solution particles of the present invention. Therefore, each catalyst has excellent catalytic activity in the corresponding reaction.

### Brief Description of Drawings

[Figure 1] Figures 1a and 1b show a TEM image and the particle size distribution, respectively, of Pt•NPs obtained in Comparative Example 1. Figures 1c and 1d show a TEM image and the particle size distribution, respectively, of PtW@WO₃₋ₓ•NPs obtained in Example 1.
[Figure 2] Figure 2 illustrates the analytical results of PtW@WO₃₋ₓ•NPs obtained in Example 1. Figures 2a-d are images showing the results of elemental mapping of Pt and W (core: Pt, shell: W). Figure 2e is a line profile chart of Pt or W. Figure 2f is charts each indicating an XRD pattern (top: Pt•NPs, bottom: PtW@WO₃₋ₓ•NPs).
[Figure 3] Figure 3 is spectrograms of PtW@WO₃₋ₓ as measured by XPS (left: W4f, right: Pt4f).
[Figure 4] Figure 4a is a TEM image of Pt•NPs/C (on carbon) obtained in Comparative Example 2. Figure 4b is a TEM image of PtW@WO₃₋ₓ•NPs/C (on carbon) obtained in Example 1.
[Figure 5] Figures 5a-d are images showing the results of elemental mapping of PtW@WO₃₋ₓ•NPs/C obtained in Example 1.
[Figure 6] Figure 6 illustrates the analytical results of PtW@WO₃₋ₓ•NPs/C obtained in Example 1 after EC cleaning. Figures 6a-d are images showing the results of elemental mapping of Pt and W. Figure 6e is a line profile chart of Pt or W.
[Figure 7] Figures 7a-d are images showing the analytical results of PtW•NPs obtained in Example 2 and showing the results of elemental mapping of Pt and W.
[Figure 8] Figures 8a-b show the line profiles of Pt and W of PtW•NPs obtained in Example 2.
[Figure 9] Figures 9a-c are charts each showing cyclic voltammetry (CVs). Figure 9a represents commercial (comm) - Pt•NPs/C, Figure 9b represents Pt•NPs/C, and Figure 9c represents PtW•NPs/C. Figure 9d is a graph indicating the effective surface area (ECSA) of each of the above three samples.
[Figure 10] Figures 10a-c are a chart illustrating LSV polarization curves of comm-Pt•NPs/C, Pt•NPs/C, and PtW•NPs/C (Figure 10a), a graph indicating each mass activity (Figure 10b), and a graph indicating each specific activity (Figure 10c).
[Figure 11] Figure 11 is a chart indicated by i-t curves at a PtW•NPs/C electrode, a comm-Pt•NPs/C electrode, and a Pt•NPs/C electrode.
[Figure 12] Figure 12 shows linear sweep voltammetry (LSV) of Pt•NPs/C or Pt•NPs/C+WO₃₋ₓ.
[Figure 13] Figure 13 illustrates the analytical results of PtW@WO₃₋ₓ•NPs obtained in Example 1 after being supported on alumina. Figures 13a and 13c-e are images showing the results of elemental mapping of Pt and W. Figure 13b is a line profile chart of Pt or W.
[Figure 14] Figure 14a is a graph indicating each CO conversion. Figure 14b is a graph showing the kind and percentage of each product.
[Figure 15] Figure 15 is charts indicating XRD patterns before and after a CO hydrogenation reaction. Figure 15a shows the results of PtW•NPs/Al₂O₃, and Figure 15b shows the results of Pt•NPs/Al₂O₃.
[Figure 16] Figure 16 shows TEM images and the particle size distributions before and after the CO hydrogenation reaction. Figure 16a shows the results of PtW•NPs/Al₂O₃. Figure 16b shows the results of Pt•NPs/Al₂O₃.
[Figure 17] Figure 17a is a graph indicating each CO₂ conversion, and Figure 17b is a graph showing the kind and percentage of each product.
[Figure 18] Figure 18 is charts indicating XRD patterns before and after a CO₂ hydrogenation reaction. Figure 18a shows the results of PtW•NPs/Al₂O₃. Figure 18b shows the results of Pt•NPs/Al₂O₃.
[Figure 19] Figure 19 shows TEM images and the particle size distributions before and after the CO₂ hydrogenation reaction. Figure 19a shows the results of PtW•NPs/Al₂O₃. Figure 19b shows the results of Pt•NPs/Al₂O₃.
[Figure 20] Figure 20 is a graph showing the results of measuring each CO₂ conversion in Example 6.
[Figure 21] Figure 21 is a graph showing the results of measuring each CO₂ conversion in Comparative Example 9.
[Figure 22] Figure 22 is charts indicating the X-ray diffraction patterns and crystallite sizes in Example 6 or Comparative Example 9.
[Figure 23] Figure 23 illustrates the measurement results in Example 7. Figure 23(a) is a chart showing each X-ray diffraction pattern. Figure 23(b) is a graph indicating each crystallite size.
[Figure 24] Figure 24 illustrates the measurement results in Comparative Example 10. Figure 24(a) is a chart showing each X-ray diffraction pattern. Figure 24(b) is a graph indicating each crystallite size.

### Mode of Carrying Out the Invention

The particles of the present invention are platinum-tungsten solid solution particles comprising platinum and tungsten in solid solution at an atomic level. Here, the term "platinum-tungsten solid solution particles" refers to solid solution particles of platinum and tungsten. The solid solution means a state in which constituent elements are mixed at an atomic level. The solid solution is one of forms included in the concept of alloy. The case of alloy typically includes not only a solid solution type alloy but also a non-solid-solution-based alloy. Platinum and tungsten, which are constituent elements of particle in the present invention, are in solid solution at an atomic level to form a solid solution. The particle size of the platinum-tungsten solid solution particles of the present invention is not particularly limited. However, from the viewpoint of improving the activity as a catalyst, the range of average particle size from 1 to 20 nm, 1 to 10 nm, or the like may be suitably exemplified. When the average particle size is in the above range, the catalytic activity can be further improved due to the high surface area. The tungsten content ratio in the platinum-tungsten solid solution particles of the present invention is not particularly limited, but a range of 1 to 50 atomic percent or the like may be suitably exemplified. When the tungsten content ratio is in the above range, the catalytic activity can be further improved due to the negative charge of (negatively charged) Pt.

A method for producing platinum-tungsten solid solution particles according to the present invention is exemplified. The solid solution particles of the present invention may be produced, for example, by a pyrolysis process using liquid phase synthesis. In the pyrolysis process, a solution containing a solvent, a platinum compound, and a tungsten compound is kept at a predetermined temperature such as 300°C or higher for a predetermined time such as 1 hour or longer. This allows for production of the solid solution particles of the present invention. As the solvent, the following or the like may be exemplified: an ether-based solvent (e.g., diphenyl ether, dibenzyl ether, ditolyl ether, 1,2-diphenoxyethane, ethylene glycol monophenyl ether, diethylene glycol monophenyl ether, ethylene glycol, diethylene glycol, propylene glycol, polyethylene glycol, polypropylene glycol, triethylene glycol, tetraethylene glycol, ethylene glycol monobutyl ether, diethylene glycol monomethyl ether, diethylene glycol monoethyl ether, diethylene glycol monobutyl ether, diethylene glycol diethylene ether, dipropylene glycol monomethyl ether, dipropylene glycol dimethyl ether); higher fatty acid (e.g., linoleic acid, linolenic acid, palmitic acid, stearic acid); primary amine (e.g., alkyl amine (e.g., laurylamine, myristylamine, cetylamine, stearylamine, behenylamine), oleylamine); unsaturated fatty acid (e.g., oleic acid); a glycol compound (e.g., propylene glycol, diethylene glycol), which are each a high-boiling-point solvent. Two or more kinds thereof may be mixed and used. In the pyrolysis process, the boiling point of the solvent is preferably 300°C or higher and more preferably 330°C or higher. As the platinum compound, a platinum compound (e.g., bis(acetylacetonato)platinum, platinum chloride, hexachloroplatinic acid salt, dichloro(1,5-cyclooctadiene)platinum (II), di-µ-chlorobis[chloro(cyclohexene)platinum], dimethyl(1,5-cyclooctadiene)platinum) or the like may be exemplified. As the tungsten compound, tungsten carbonyl, tungsten chloride, bis(cyclopentadienyl)tungstendichloride, bis(ethylcyclopentadienyl)tungsten(IV) dichloride, bis(isopropylcyclopentadienyl)tungsten(IV) dichloride, bis(cyclopentadienyl)tungsten(IV) dihydride, bis(isopropylcyclopentadienyl)tungsten(IV) dihydride or the like may be exemplified.

As another example of the method for producing platinum-tungsten solid solution particles according to the present invention, a reduction process is exemplified. In the reduction process, a platinum compound and a tungsten compound are dissolved in a solvent, and a reducing agent is added and preferably stirred to be able to produce the solid solution particles of the present invention. The suspension after the reducing agent is added and retained or stirred may be centrifuged, washed, and dried to produce the solid solution particles of the present invention. As long as the raw material can be dissolved, as the solvent that can be used, the following or the like may be exemplified: in addition to the above described high-boiling-point solvents, an alcohol compound (e.g., methanol, ethanol, propanol); aromatic hydrocarbon (e.g., benzene, toluene); halogenated hydrocarbon (e.g., methylene chloride, chloroform); acetone, ethyl acetate, acetonitrile, N,N-dimethylformamide (DMF), N,N-diethylformamide, N,N-dimethylacetamide, diethyl ether, 1,2-dimethoxyethane, tetrahydrofuran (THF). Two or more kinds thereof may be mixed and used. As the platinum compound, a platinum compound (e.g., bis(acetylacetonato)platinum, platinum chloride, hexachloroplatinic acid salt, dichloro(1,5-cyclooctadiene)platinum (II), di-µ-chlorobis[chloro(cyclohexene)platinum], dimethyl(1,5-cyclooctadiene)platinum) or the like may be exemplified. As the tungsten compound, tungsten carbonyl, tungsten chloride, bis(cyclopentadienyl)tungsten dichloride, bis(ethylcyclopentadienyl)tungsten(IV) dichloride, bis(isopropylcyclopentadienyl)tungsten(IV) dichloride, bis(cyclopentadienyl)tungsten(IV) dihydride, bis(isopropylcyclopentadienyl)tungsten(IV) dihydride or the like may be exemplified. As the reducing agent, the following or the like may be exemplified: a borane compound (e.g., a borane-ammonia complex, a borane-tert-butylamine complex, a borane-dimethylamine complex, a borane-diisopropylamine complex, a borane-dicyclohexylamine complex, a borane-trimethylamine complex, a borane-triethylamine complex, a borane-N,N-diethylaniline complex, a borane-pyridine complex, a borane-2-picoline complex, a borane-morpholine complex, a borane-tetrahydrofuran complex, a borane-dimethyl sulfide complex, 1,2-bis(tert-butylthio)ethane borane, a borane-triphenylphosphine complex); a borohydride compound (e.g., sodium borohydride, lithium borohydride, potassium borohydride, sodium borohydride triacetate, sodium cyanoborohydride, sodium tris(1,1,1,3,3,3-hexafluoroisopropoxy)borohydride, lithium triethylborohydride, potassium dihydrobis(1-pyrazolyl)borate, potassium dihydrobis(1-pyrazolyl)borate, potassium tris(1-pyrazolyl)borohydride, (polystyrylmethyl)trimethylammonium cyanoborohydride, tetramethylammonium hydroxide, tetramethylammonium borohydride, benzyltriethylammonium borohydride, tetrabutylammonium borohydride, tetramethylammonium triacetoxyborohydride); an aluminum hydride compound (e.g., lithium aluminum hydride); a metal hydride compound; a silane compound; or a hydrazine compound.

The catalyst of the present invention is characterized by containing the platinum-tungsten solid solution particles of the present invention. The catalyst of the present invention may be utilized as a catalyst for various reactions such as hydrogen evolution, carbon monoxide hydrogenation, carbon dioxide hydrogenation, acetylene hydrogenation, ethylene hydrogenation, formation of CO₂ from methane, aqueous gas shift, CO oxidation, and/or dehydrogenation (e.g., synthesis of benzene from cyclohexane) . When used as a catalyst, the platinum-tungsten solid solution particles of the present invention may be supported on a carrier. By allowing the particles to be supported on a carrier, the particles can be prevented from aggregation during the catalytic reaction. The carrier is not particularly limited, and any known carrier may be used. As the carrier, a carbon (C), alumina (Al₂O₃), titanium dioxide (TiO₂), magnesium oxide (MgO), cerium dioxide (CeO₂), or praseodymium oxide (Pr₆O₁₁) particle or the like may be exemplified. Alternatively, the platinum-tungsten solid solution particles of the present invention may be used as a catalyst without being supported on a carrier. When the solid solution particles are used as a catalyst in solution, they may be protected with a protective agent such as poly(N-vinyl-2-pyrrolidone) (PVP). The platinum-tungsten solid solution particles of the present invention exhibit excellent catalytic activity as well as durability when used as a catalyst. As the catalyst of the present invention, the platinum-tungsten solid solution particles of the present invention may be used alone or may be mixed with other component (s) as long as the catalytic activity is not inhibited or is further improved. As a preferred embodiment of the catalyst of the present invention, a hydrogen evolution catalyst comprising the platinum-tungsten solid solution particles of the present invention, a carbon monoxide hydrogenation catalyst comprising the platinum-tungsten solid solution particles of the present invention, a carbon dioxide hydrogenation catalyst comprising the platinum-tungsten solid solution particles of the present invention or the like may be exemplified. The method for producing the catalyst of the present invention is not particularly limited. However, in the case where the solid solution particles of the present invention are supported on a carrier, for example, a suspension of the solid solution particles of the present invention and the carrier may be prepared, heated, cooled, and then dried to produce the catalyst.

Hereinafter, the present invention will be described in detail with reference to Examples of the present invention. However, the technical scope of the present invention is not limited to these Examples.

### [Example 1]

### (Preparation of PtW-WO₃₋ₓ Core-Shell Structure)

In a 100-mL three-necked flask, 60 mL of oleylamine was added, and the temperature was raised to 100°C under vacuum. Then, 7.5 mmol of tungsten carbonyl and 1.5 mmol of bis(acetylacetonato)platinum were added, and the temperature was raised to 330°C under nitrogen. The mixture was stirred for 1 hour. After stirring, the mixture was cooled to room temperature. The resulting black solution was washed several times with a mixed solution of ethanol and hexane (ethanol:hexane = 1:1) . After washing, the resulting material was centrifuged to prepare black powder. The resulting black powder is denoted as PtW@WO₃₋ₓ•NPs (NPs means nanoparticles).

### (Preparation of PtW@WO₃-ₓ•NPs on Carbon)

First, 25 mL of hexane, 0.02 mL of oleylamine, and 33.8 mg of PtW@WO₃₋ₓ•NPs obtained in Example 1 were sonicated in a 50-mL flask for 30 minutes to give a suspension. Subsequently, a suspension obtained by adding 67.3 mg of carbon (Valcan (R) XC72R) to 15 mL of 2-propanol was added to the above suspension, and the mixture was sonicated for 2 hours. After evaporation of hexane and 2-propanol, 40 mL of acetic acid was added, and the mixture was heated in an oil bath at 70°C for 15 hours. The reaction mixture was cooled to room temperature and centrifuged. The obtained black powder was washed several times with a mixture of ethanol and acetone, and finally centrifuged to prepare black powder. The resulting black powder is denoted as PtW@WO₃₋ₓ•NPs/C.

### (Preparation of Pt-W Solid Solution)

Here, 75 cycles of electrochemical cleaning (EC cleaning) in the range of -0.15 V to 0.9 V (vs. Ag/AgCl) were performed on PtW@WO₃₋ₓ•NPs/C.

### [Comparative Example 1]

### (Preparation of Pt Nanoparticles)

In a 100-mL three-necked flask, 60 mL of oleylamine was added, and the temperature was raised to 100°C under vacuum. Then, 1.5 mmol of bis(acetylacetonato)platinum was added, and the temperature was raised to 330°C under nitrogen. The mixture was stirred for 1 hour. After stirring, the mixture was cooled to room temperature. The resulting black solution was washed several times with a mixed solution of ethanol and hexane (ethanol:hexane = 1:1). After washing, the resulting material was centrifuged to prepare black powder. The resulting black powder is denoted as Pt•NPs (Pt nanoparticles).

### (Preparation of Pt•NPs on Carbon)

First, 25 mL of hexane, 0.02 mL of oleylamine, and 28.5 mg of Pt•NPs obtained in Comparative Example 1 were sonicated in a 50-mL flask for 30 minutes to give a suspension. Subsequently, a suspension obtained by adding 66.1 mg of carbon (Valcan (R) XC72R) to 15 mL of 2-propanol was added to the above suspension, and the mixture was sonicated for 2 hours. After evaporation of hexane and 2-propanol, 40 mL of acetic acid was added, and the mixture was heated using an oil bath at 70°C for 15 hours. The reaction mixture was cooled to room temperature and centrifuged. The obtained black powder was washed several times with a mixture of ethanol and acetone, and finally centrifuged to prepare black powder. The resulting black powder is denoted as Pt•NPs/C. EC cleaning was also performed on Pt•NPs/C in substantially the same manner as for PtW@WO₃₋ₓ•NPs/C.

### (Analysis of PtW@WO₃-ₓ•NPs or Pt•NPs)

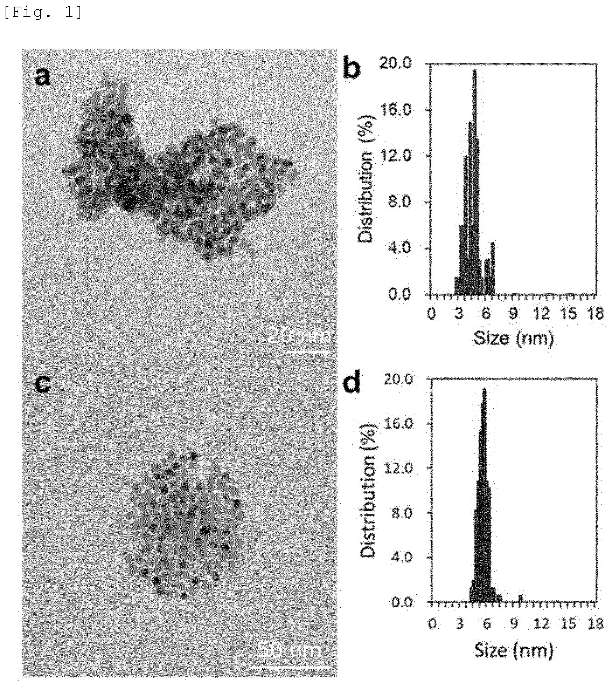

PtW@WO₃-ₓ•NPs obtained in Example 1 and Pt•NPs obtained in Comparative Example 1 were observed by transmission electron microscopy (TEM). Figure 1 shows TEM images and the particle size distributions obtained from the TEM images. Figures 1a and 1b are a TEM image and the particle size distribution of Pt•NPs, and Figures 1c and 1d are a TEM image and the particle size distribution of PtW@WO₃₋ₓ•NPs. The average particle size of PtW@WO₃-ₓ•NPs was 5.5 ± 0.6 nm, and that of Pt•NPs was 4.4 ± 0.9 nm. The "A" in the notation "A ± B nm" represents the average particle size and the "B" represents the standard deviation. While the particle size used is the long diameter of particle in a TEM image, the average particle size was calculated by measuring the particle sizes (long diameters) of particles (at least 300 particles) in a TEM image and then averaging the measured diameters.

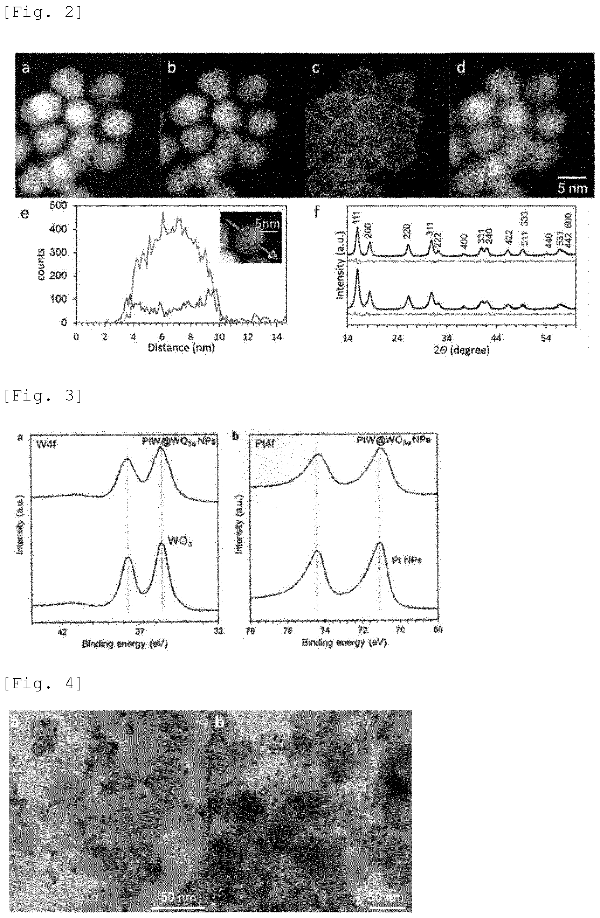

The atomic ratio between Pt and W (Pt:W) in PtW@WO₃₋ₓ•NPs was determined by inductively coupled plasma optical emission spectrometry (ICP-OES) to be 74:26. To check the structure of PtW@WO₃₋ₓ•NPs, elemental mapping and line analysis were performed by energy dispersive X-ray analysis (EDX). A high-angle annular dark-field scanning transmission electron microscopy (HAADF-STEM) image and elemental mapping results are shown in Figures 2a-e. Figure 2a shows the HAADF-STEM image, Figure 2b shows the results of elemental mapping of Pt (green), Figure 2c shows the results of elemental mapping of W (red), and Figure 2d shows the results obtained by superimposing the results of Figures 2b and 2c. The outer points (red) of each particle in Figure 2d each represent W in the image of Figure 2c, and the inner points (green) each represent Pt in the image of Figure 2b. Figure 2e is a chart showing the line profiles of Pt and W recorded along the arrow in the HAADF-STEM image at the upper right in the chart. The upper broken line (green) represents Pt and the lower broken line (red) represents W. Figure 3 shows the spectra measured by X-ray photoelectron spectroscopy (XPS). Figure 3a shows the 4f peaks of W. The upper broken line shows PtW@WO₃₋ₓ•NPs, and the lower broken line shows WO₃. Figure 3b shows the 4f peaks of Pt. The upper broken line shows PtW@WO₃₋ₓ•NPs, and the lower broken line shows Pt•NPs. From Figures 2a-e and 3, it can be seen that PtW@WO₃₋ₓ•NPs has a core-shell structure with a WO₃₋ₓ shell and a core containing Pt and W.

Figure 2f shows the XRD patterns of PtW@WO₃₋ₓ•NPs (upper line: blue) and Pt•NPs (lower line: red) by radiation X-ray diffraction (XRD) . PtW@WO₃₋ₓ•NPs formed a single-component fcc structure similar to that of Pt•NPs. The reason why diffraction peaks of WO₃₋ₓ shell are not observed is considered to be that WO₃₋ₓ is amorphous and/or a thin layer. The lattice constants of PtW@WO₃₋ₓ•NPs and Pt•NPs were evaluated to be 3.922 Å and 3.920 Å, respectively, by Le Bail fitting of XRD patterns. The reason why there is no obvious difference in the lattice constant between PtW@WO₃₋ₓ•NPs and Pt•NPs is that Pt and W atoms have almost the same metallic bond radius.

### (Analysis of PtW@WO₃₋ₓ•NPs/C)

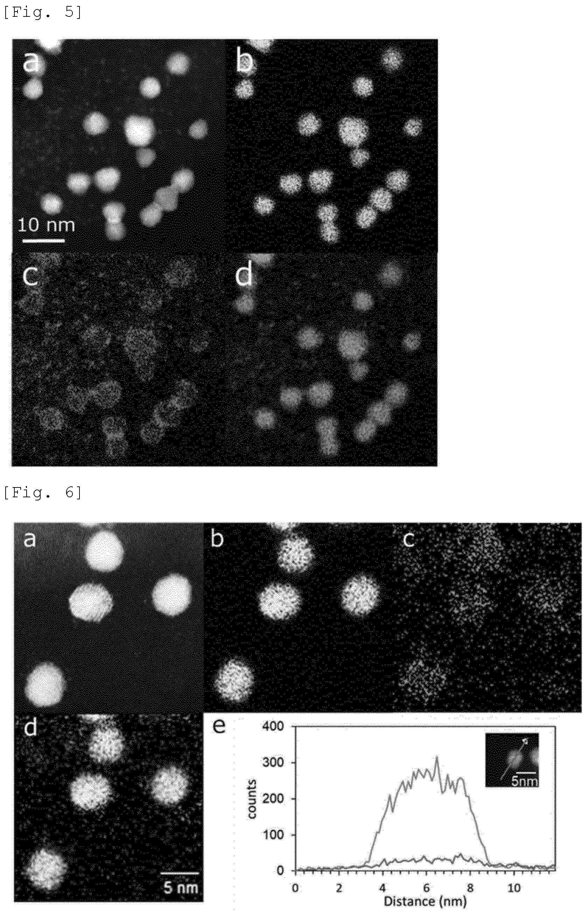

Figure 4a shows a TEM image of Pt•NPs/C and Figure 4b shows a TEM image of PtW@WO₃₋ₓ•NPs/C. As can be seen from Figures 4a and 4b, PtW@WO₃₋ₓ•NPs/C was well dispersed on the carbon carrier like in the case of Pt•NP/C. Figure 5 shows a HAADF-STEM image and the elemental mapping results of PtW@WO₃₋ₓ•NPs/C. Figure 5a shows the HAADF-STEM image, Figure 5b shows the results of elemental mapping of Pt (green), Figure 5c shows the results of elemental mapping of W (red), and Figure 5d shows the results obtained by superimposing the results of Figures 5b and 5c. The outer points (red) of each particle in Figure 5d each represent W in the image of Figure 5c, and the inner points (green) each represent Pt in the image of Figure 5b. As can be seen from Figures 5a-d, the core-shell structure was maintained after the particles are supported on carbon.

### (Analysis of PtW@WO₃₋ₓ•NPs/C After EC Cleaning)

Figures 6a-e show a HAADF-STEM image and the results of elemental mapping after EC cleaning. Figure 6a shows the HAADF-STEM image, Figure 6b shows the results of elemental mapping of Pt (green), Figure 6c shows the results of elemental mapping of W (red), and Figure 6d shows the results obtained by superimposing the results of Figures 6b and 6c. Figure 6e is a chart showing the line profiles of Pt and W recorded along the arrow in the HAADF-STEM image at the upper right in the chart. The upper broken line (green) represents Pt and the lower broken line (red) represents W. In Figure 6d, W at the periphery of Pt as in Figure 2d is absent. In Figure 6e, the presence of W at the periphery of Pt as in Figure 2e was also not observed. Thus, the WO₃₋ₓ shell layer disappeared after EC cleaning. In addition, Figure 6e also shows that Pt and W are randomly and uniformly distributed in each nanoparticle. Particles in which the WO₃₋ₓ shell layer disappears from PtW@WO₃₋ₓ•NPs are denoted as PtW•NPs, and PtW•NPs/C is denoted including carbon as a carrier. The percentage of W in PtW•NPs was measured to be 4 atomic percent (at%) by STEM/EDS line analysis. In addition, XPS measurements of PtW@WO₃₋ₓ (Figure 3) demonstrated a shift with respect to Pt•NPs. The Pt-4f peak measured by XPS was shifted from 71.20 eV to 71.06 eV. The HAADF-STEM image and elemental mapping results indicate that the above measured peaks originate from the core, since the shell layer is free of Pt. Since Pt and W have some difference in electronegativity, in the case of a solid solution of Pt and W, the charge transfer causes a shift of the peaks measured by XPS. The results in Figure 3 indicate that Pt is negatively charged in the core because electrons are injected from W to Pt, supporting that the core (i.e., PtW•NPs) is a solid solution. These results have revealed that the core of PtW@WO₃-ₓ•NPs is a solid solution in which Pt and W are in solid solution at an atomic level.

### [Example 2]

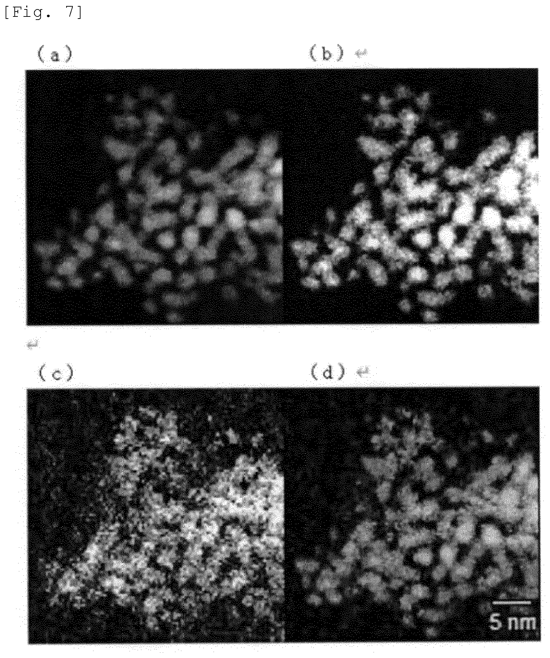

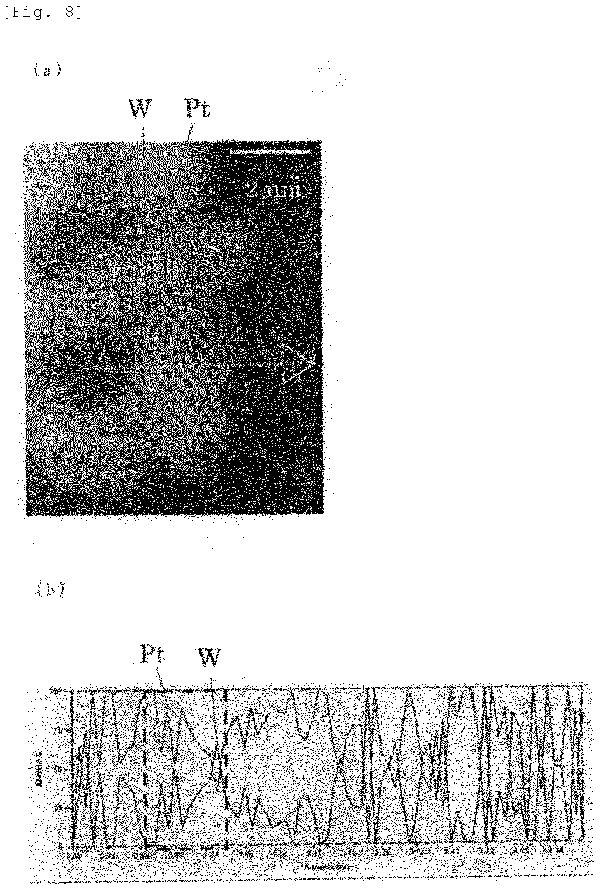

To a mixed solvent containing 8.3 mL of dibenzyl ether, 1.7 mL of oleylamine, and 1.0 mL of oleic acid were added 0.15 mmol of bis(cyclopentadienyl)tungsten dichloride and 0.15 mmol of dichloro(1,5-cyclooctadiene)platinum (II) to give a suspension. The temperature was raised to 130°C and the suspension was then dissolved. To this was added 2.2 mmol of borane-morpholine complex. The mixture was stirred for 30 minutes, centrifuged, and washed (with a mixed solvent of hexane and ethanol) repeatedly. After vacuum drying, 42.7 mg of black powder was then obtained. Elemental mapping and line analysis by energy dispersive X-ray analysis (EDX) of the black powder obtained in substantially the same manner as in Example 1 were performed. Figures 7(a) to 7(d) show a high-angle annular dark-field scanning transmission electron microscopy (HAADF-STEM) image and the elemental mapping results. Figure 7a shows the HAADF-STEM image, Figure 7b shows the results of elemental mapping of Pt (green), Figure 7c shows the results of elemental mapping of W (red), and Figure 7d shows the results obtained by superimposing the results of Figures 7b and 7c. Figure 8 shows the results of EDX line analysis at the atomic resolution level. Figures 8(a) and 8(b) show the results of the line analysis in the direction of the arrow of Figure 8 (a). Figure 8(b) has demonstrated the presence of 50 atomic% tungsten locally inside the particles. The green line represents Pt and the red line represents W. In addition, X-ray fluorescence (XRF) analysis showed that the ratio of the number of Pt atoms to the number of W atoms in the whole particle (Pt:W) was 76:24. Figures 7(b) and 7(c) shows that Pt and W are distributed in the same position, suggesting that Pt and W are in solid solution. In Example 2, solid solution particles comprising Pt and W in solid solution (PtW•NPs) without a core-shell structure were obtained.

### [Example 3]

A mixture (with 16 mass% of Pt) containing 0.65 mL of 2-propanol, 0.05 mL of Nafion, 0.3 mL of hexane, and PtW•NPs/C obtained in Example 1 were sonicated for 30 minutes to prepare ink for application of PtW•NPs/C to an electrode. Then, 5 µL (0.8 mgPt/mL) of the resulting ink was applied to a rotating disk electrode (working electrode; manufactured by BAS, Inc.), and dried under air.

### [Comparative Example 2]

### (To Prepare Electrode)

Ink was prepared in the same manner as in Example 3 except that PtW•NPs/C was replaced by Pt•NPs/C. The ink was applied to a rotating disk electrode, so that the amount of Pt was the same as in Example 3, and dried under air.

### [Comparative Example 3]

### (Commercially Available Pt on Carbon)

Commercially available Pt on carbon (Pt loading of 20 mass%; manufactured by Alfa Aesar) was prepared. This is denoted as commercial(comm)-Pt•NPs/C.

### (To Prepare Electrode)

Ink was prepared in the same manner as in Example 3 except that PtW•NPs/C was replaced by comm-Pt•NPs/C. The ink was applied to a rotating disk electrode, so that the amount of Pt was the same as in Example 3, and dried under air.

### [Comparative Example 4]

### (Preparation of WO₃₋ₓ Cluster)

In a 50-mL three-necked flask, 20 mL of oleylamine was added, and the temperature was raised to 100°C under vacuum. Then, 2.5 mmol of tungsten carbonyl was added, and the temperature was raised to 330°C under nitrogen. The mixture was stirred for 1 hour. After stirring, the mixture was cooled to room temperature. The resulting black solution was washed several times with a mixed solution of ethanol and hexane (ethanol:hexane = 1:1) . After washing, the resulting material was centrifuged to prepare black powder.

### (Preparation of Mixture of Pt•NPs/C and WO₃₋ₓ Cluster)

Here, 20.0 mg of Pt•NPs/C obtained in Comparative Example 1 and 1.4 mg of the above-obtained WO₃₋ₓ cluster were mixed to prepare a mixture. The resulting mixture is denoted as Pt•NPs/C+WO₃₋ₓ.

### (To Prepare Electrode)

Ink was prepared in the same manner as in Example 3 except that PtW•NPs/C was replaced by Pt•NPs/C+WO₃₋ₓ. The ink was applied to a rotating disk electrode, so that the amount of Pt was the same as in Example 3, and dried under air.

### (Evaluation as HER Catalyst)

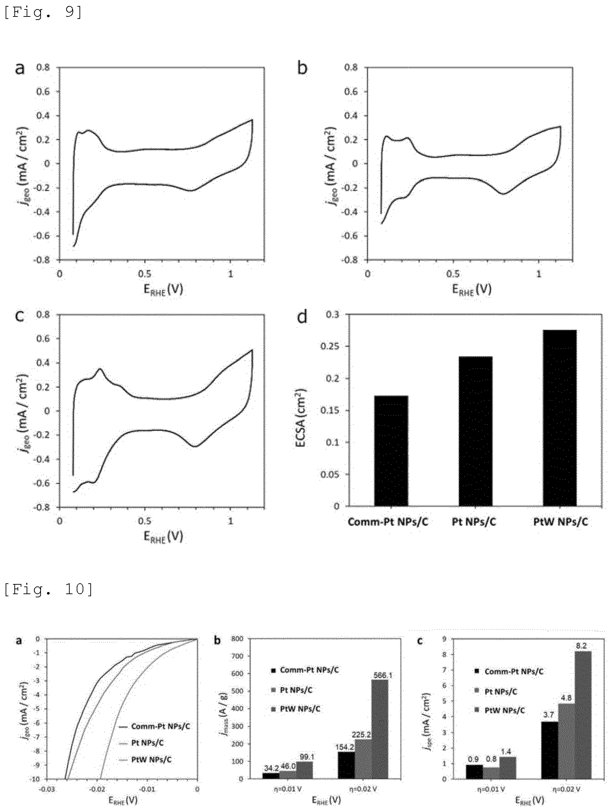

Cyclic voltammetry (CVs) was conducted using Ag/AgCl, Pt wire, and 0.5 M H₂SO₄ as a reference electrode, a counter electrode, and an electrolyte, respectively (Figures 9a-c). Figure 9a represents comm-Pt•NPs/C, Figure 9b represents Pt•NPs/C, and Figure 9c represents PtW•NPs/C. In addition, Figure 9d shows the electrochemically active surface area (effective surface area: ECSA) calculated from the CVs. The ECSA of PtW•NPs/C was larger than that of comm-Pt•NPs/C or Pt•NPs/C. Figure 10a shows the LSV polarization curves for comm-Pt•NPs/C (upper line), Pt•NPs/C (middle line), and PtW•NPs/C (lower line). The overvoltage of PtW•NPs/C was 19.4 mV at a current density of 10 mA/cm², which was much lower than that of comm-Pt•NPs/C (26.3 mV) or Pt•NPs/C (25.9 mV). The hydrogen evolution half-reaction in acidic solution is expressed by 2H⁺ + 2e⁻ → H₂. In other words, at a certain potential, the more the current (electrons), the more hydrogen is generated and the more active the electrode is. In addition, Figure 10b shows the Pt mass activity at either η of 0.01 V or 0.02 V. The mass activity of comm-Pt•NPs/C is shown on the left, that of Pt•NPs/C is shown in the middle, and that of PtW•NPs/C is shown on the right at each voltage. The mass activity of PtW•NPs/C was 99.1 A/g or 566.1 A/g at η of 0.01 V or 0.02 V, respectively, which was 2.9 times or 3.7 times higher than that of comm-Pt•NPs/C, respectively. Also, the mass activity was 2.2 times or 2.5 times that of Pt•NPs/C. Figure 10c shows the Pt specific activity at either η of 0.01 V or 0.02 V. The activity per effective unit area of comm-Pt•NPs/C is shown on the left, that of Pt•NPs/C is shown in the middle, and that of PtW•NPs/C is shown on the right at each voltage. The specific activity of PtW•NPs/C was 1.4 mA/cm² or 8.2 mA/cm² at η of 0.01 V or 0.02 V, respectively, which was 1.6 times or 2.2 times higher than that of comm-Pt•NPs/C, respectively. Also, the specific activity was 1.8 times or 1.7 times that of Pt•NPs/C, respectively. Figures 10a-c have revealed that the activity per effective unit area and the activity per mass of PtW•NPs/C are much higher than those of comm-Pt•NPs/C or Pt•NPs/C. This means that PtW•NPs/C has an ability to generate hydrogen from water at small potentials. Specifically, this indicates that PtW•NPs/C has higher HER activity than comm-Pt•NPs/C or Pt•NPs/C.

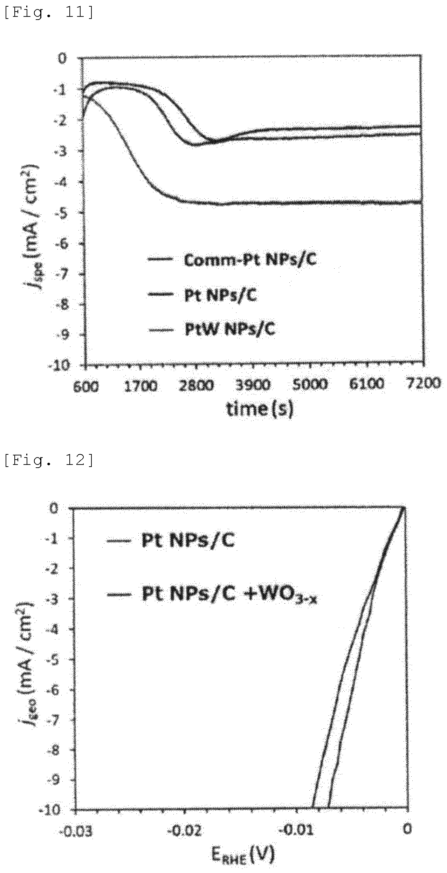

In addition, a voltage of 100 mV was applied over 2 hours to a PtW•NPs/C electrode, a comm-Pt•NPs/C electrode, or a Pt•NPs/C electrode to evaluate their stability. Ag/AgCl, a carbon rod, and 0.5 M H₂SO₄ were used as a reference electrode, a counter electrode, and an electrolyte, respectively. Figure 11 shows the results as i-t curves. The comm-Pt•NPs/C, Pt•NPs/C, and PtW•NPs/C are in the order such that the jₛₚₑ at 7200 seconds is closer to 0. It was found that the current density of PtW•NPs/C remained approximately 2.1 times higher than that of comm-Pt•NPs/C or Pt•NPs/C even after continuous application of potential for 2 hours. PtW•NPs was demonstrated to be highly stable under acidic conditions. To further confirm the effect of PtW•NPs/C, the results of linear sweep voltammetry (LSV) measurements of Pt•NPs/C and Pt•NPs/C+WO₃₋ₓ are shown in Figure 12. Pt•NPs/C and Pt•NPs/C+WO₃₋ₓ are in the order such that the j _{geo} at -10 is closer to 0. As shown in Figure 12, the activity of Pt•NPs/C+WO₃₋ₓ is similar to that of Pt•NPs/C. This indicates that PtW•NPs/C, which is a solid solution comprising Pt and W in solid solution at an atomic level, exhibits much higher activity than Pt•NPs/C+WO₃₋ₓ, which has been mechanically mixed with WO₃₋ₓ.

### [Example 4]

### (Preparation of PtW•NPs on Alumina)

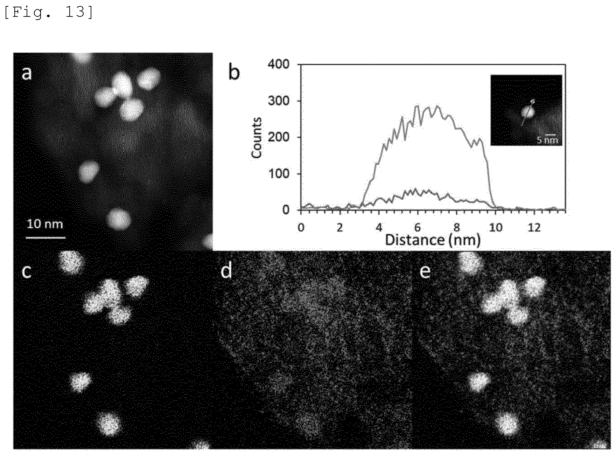

PtW@WO₃₋ₓ•NPs obtained in Example 1 was supported on alumina (AKP-G15, manufactured by Sumitomo Chemical Co., Ltd.; γ-alumina) by the following process. To a 110-mL vial were added 16.7 mg of PtW@WO₃₋ₓ•NPs, 25 mL of hexane, and 1.0 mL of oleylamine. The mixture was subjected to sonication for 30 minutes. Subsequently, 30 mL of 2-propanol suspension containing 183.3 mg of γ-alumina, which had been previously sonicated for 30 minutes, was added to the above 110-mL vial, and the mixture was sonicated for another 1 hour. The target material recovered by centrifugation was washed several times with a mixture of hexane and ethanol, and then further washed with acetone. The recovered powder was inspected. In the process of allowing PtW@WO₃₋ₓ•NPs to be supported on alumina, WO₃₋ₓ in the shell was peeled off and disappeared, and platinum-tungsten solid solution particles were found to be supported on alumina. The resulting particles having the platinum-tungsten solid solution particles supported on alumina are denoted as PtW•NPs/Al₂O₃. Figures 13a-e show a HAADF-STEM image and the results of elemental mapping after the particles are supported on alumina. Figure 13a shows the HAADF-STEM image, Figure 13c shows the results of elemental mapping of Pt (green), Figure 13d shows the results of elemental mapping of W (red), and Figure 13e shows the results obtained by superimposing the results of Figures 6c and 6d. Figure 13b is a chart showing the line profiles of Pt and W recorded along the arrow in the HAADF-STEM image at the upper right in the chart. The upper broken line (green) represents Pt and the lower broken line (red) represents W. In Figure 13e, W at the periphery of Pt as in Figure 2d is absent. In Figure 13b, the presence of W at the periphery of Pt as in Figure 2e was also not observed. Thus, the WO₃₋ₓ shell layer disappeared after the particles were supported on alumina.

### [Comparative Example 5]

### (Preparation of Pt•NPs on Alumina)

Pt•NPs obtained in Comparative Example 1 were supported on the same alumina as in Example 4 by the following process. To a 110-mL vial were added 12.2 mg of Pt•NPs, 25 mL of hexane, and 1.0 mL of oleylamine. The mixture was subjected to sonication for 30 minutes. Subsequently, 30 mL of 2-propanol suspension containing 187.8 mg of γ-alumina, which had been previously sonicated for 30 minutes, was added to the above 110-mL vial, and the mixture was sonicated for another 1 hour. The target material recovered by centrifugation was washed several times with a mixture of hexane and ethanol, and then further washed with acetone. The resulting particles having Pt•NPs supported on alumina are denoted as Pt•NPs/Al₂O₃.

### [Comparative Example 6]

### (Preparation of (Pt•NPs+WO₃₋ₓ) on Alumina)

Pt•NPs and WO₃₋ₓ were supported on the same alumina as in Example 4 by the following process. To a 110-mL vial were added 12.2 mg of Pt•NPs, 10.4 mg of WO₃₋ₓ cluster, 25 mL of hexane, and 1.0 mL of oleylamine. The mixture was subjected to sonication for 30 minutes. Subsequently, 30 mL of 2-propanol suspension containing 187.8 mg of γ-alumina, which had been previously sonicated for 30 minutes, was added to the above 110-mL vial, and the mixture was sonicated for another 1 hour. The target material recovered by centrifugation was washed several times with a mixture of hexane and ethanol, and then further washed with acetone. The resulting particles having Pt•NPs and WO₃₋ₓ supported on alumina are denoted as (Pt•NPs+WO₃₋ₓ) /Al₂O₃.

### (CO Hydrogenation Reaction)

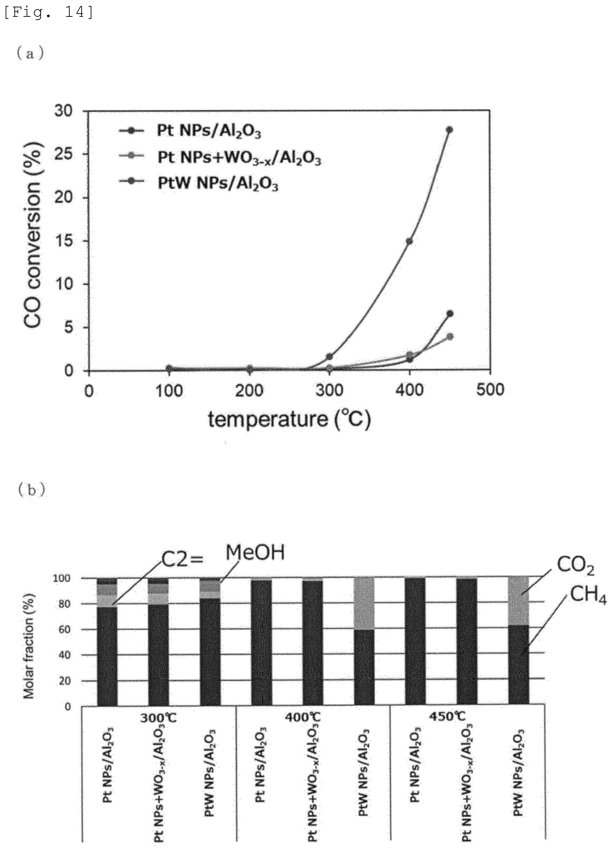

PtW•NPs/Al₂O₃ (with 3.65 mg of Pt) obtained in Example 4 was exposed to a mixed gas of Ar, CO and H₂ (Ar:CO:H₂ in the volume ratio of 20:30:10) at a pressure of 0.7 MPa and a temperature of 100°C, 200°C, 300°C, 400°C, or 450°C. The CO conversion and the converted gas species were measured by qualitative and quantitative analysis of the gas detected by gas chromatography installed at the end of the system. Figure 14 shows the results. Figure 14(a) is a graph indicating each CO conversion, and Figure 14(b) is a graph showing the kind and percentage of each product.

A test was conducted in the same manner as in the case of PtW•NPs/Al₂O₃ obtained in Example 4, except that PtW•NPs/Al₂O₃ obtained in Example 4 was replaced by Pt•NPs/Al₂O₃ obtained in Comparative Example 5. Figure 14 shows the results.

A test was conducted in the same manner as in the case of PtW•NPs/Al₂O₃ obtained in Example 4, except that PtW•NPs/Al₂O₃ obtained in Example 4 was replaced by (Pt•NPs+WO₃₋ₓ)/Al₂O₃ obtained in Comparative Example 6. Figure 14 shows the results.

In Figure 14(a), the line with the highest CO conversion at 450°C corresponds to PtW•NPs/Al₂O₃, the second highest line corresponds to Pt•NPs/Al₂O₃, and the lowest line corresponds to (Pt•NPs+WO₃₋ₓ) /Al₂O₃. As shown in Figure 14(a), PtW•NPs/Al₂O₃ exhibited CO conversion five times or higher than that of Pt•NPs/Al₂O₃ or (Pt•NPs+WO₃₋ₓ) /Al₂O₃ at, for example, 400°C, indicating a significant increase in conversion. As shown in Figure 14(b), the kinds of gas produced were similar for the case of using both catalysts at 300°C. About 80% of the product was CH₄. On the other hand, at 400°C or higher, in the case of Pt•NPs/Al₂O₃ or (Pt•NPs+WO₃₋ₓ) /Al₂O₃, majority of the product was CH₄. As in the case at 300°C, a CH₄-rich product was obtained. This suggests that the reaction of the following scheme (1) occurs in the case of Pt•NPs/Al₂O₃ or (Pt•NPs+WO₃₋ₓ) /Al₂O₃ at both low and high temperatures.

nCO + (2n+1)H₂ → CₙH₂ₙ₊₂ + nH₂O (1)

In contrast, in the case of PtW•NPs/Al₂O₃, a mixture of CH₄ and CO₂ was obtained at 400°C or higher. Therefore, in the case of PtW•NPs/Al₂O₃, the reaction in scheme (1) is considered to proceed at low temperatures, but at 400°C or higher, in addition to the reaction in scheme (1), the aqueous gas shift reaction in scheme (2) is also expected to occur due to the produced water vapor and CO, thereby resulting in CO₂. In other words, the high activity of PtW•NPs/Al₂O₃ is attributed to the predominant proceeding of the reaction in scheme (1) due to the consumption of H₂O by the aqueous gas shift reaction. The results also indicate that PtW•NPs/Al₂O₃ can be used as a catalyst for aqueous gas shift.

CO + H₂O → CO₂ + H₂ (2)

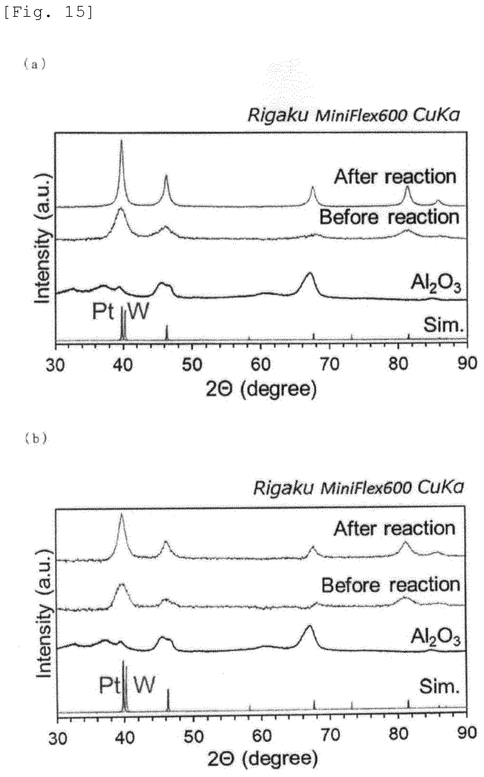

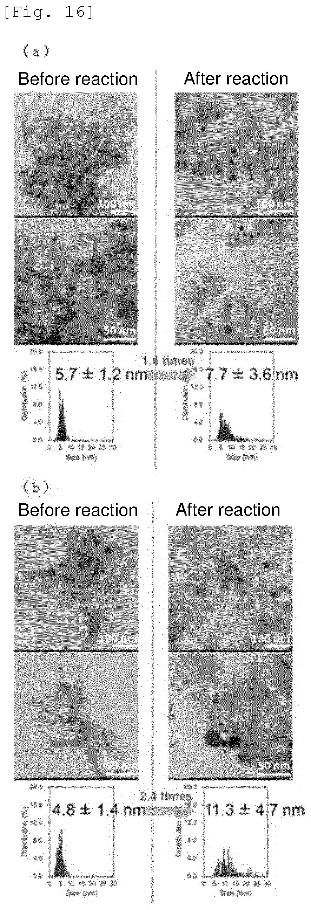

PtW•NPs/Al₂O₃ or Pt•NPs/Al₂O₃ before and after the CO hydrogenation reaction was subjected to XRD measurement. Figure 15 shows the results. Figure 15(a) shows the results of PtW•NPs/Al₂O₃, and Figure 15(b) shows the results of Pt•NPs/Al₂O₃. Note that the baseline for alumina-carrier sample was subtracted, and fitting was performed by Le Bail fitting. The results of XRD measurement has demonstrated that the X-ray diffraction peaks of any of the samples were sharpened before and after the CO hydrogenation reaction. The crystallite size was actually estimated from the fitting and compared between before and after the reaction. In the case of Pt•NPs/Al₂O₃, the size increase was 2.8-fold since the size was 5.4 nm before the reaction and 15.3 nm after the reaction. By contrast, in the case of PtW•NPs/Al₂O₃, the size increase was 1.5-fold since the size was 5.0 nm before the reaction and 7.6 nm after the reaction. In the case of PtW•NPs/Al₂O₃, an increase in the crystallite size was found to be suppressed. Further, a change in the particle size of PtW•NPs/Al₂O₃ or Pt•NPs/Al₂O₃ between before and after the CO hydrogenation reaction was observed by TEM. Figure 16 shows the results. Figure 16(a) shows the results of PtW•NPs/Al₂O₃, and Figure 16(b) shows the results of Pt•NPs/Al₂O₃. The particle size was also determined by TEM. The size of Pt•NPs/Al₂O₃ was found to be increased 2.4-fold, and the size of PtW•NPs/Al₂O₃ was found to be increased 1.4-fold. This has demonstrated that in the case of PtW•NPs/Al₂O₃, an increase in the particle size due to sintering can be suppressed.

### [Example 5]

### (CO₂ Hydrogenation Reaction)

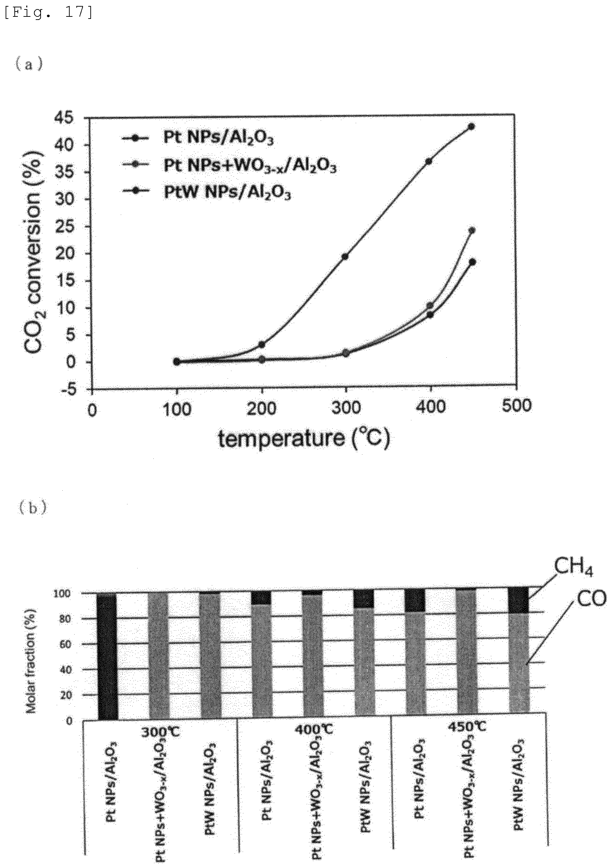

PtW•NPs/Al₂O₃ (with 3.65 mg of Pt) was exposed to a mixed gas of Ar, CO₂ and H₂ (Ar:CO₂:H₂ in the volume ratio of 20:30:10) at a pressure of 0.7 MPa and a temperature of 100°C, 200°C, 300°C, 400°C, or 450°C. The CO₂ conversion and the converted gas species were measured by qualitative and quantitative analysis of the gas detected by gas chromatography installed at the end of the system. Figure 17 shows the results. Figure 17(a) is a graph indicating each CO₂ conversion, and Figure 17(b) is a graph showing the kind and percentage of each product.

### [Comparative Example 7]

A test was conducted in the same manner as in Example 5 except that PtW•NPs/Al₂O₃ was replaced by Pt•NPs/Al₂O₃. Figure 17 shows the results.

### [Comparative Example 8]

A test was conducted in the same manner as in Example 5 except that PtW•NPs/Al₂O₃ was replaced by (Pt•NPs+WO₃-ₓ)/Al₂O₃. Figure 17 shows the results.

In Figure 17(a), the line with the highest CO₂ conversion at 450°C corresponds to PtW•NPs/Al₂O₃, the second highest line corresponds to (Pt•NPs+WO₃₋ₓ)/Al₂O₃, and the lowest line corresponds to Pt•NPs/Al₂O₃. As shown in Figure 17(a), PtW•NPs/Al₂O₃ exhibited CO₂ conversion 17 times or higher than that of Pt•NPs/Al₂O₃ or (Pt•NPs+WO₃₋ₓ) /Al₂O₃ at, for example, 300°C, indicating a significant increase in conversion. As shown in Figure 17(b), the kinds of gas produced at 300°C, namely the product was mostly CH₄ in the case of Pt•NPs/Al₂O₃. In the case of PtW•NPs/Al₂O₃, however, majority of the product was CO. The results have revealed that PtW•NPs/Al₂O₃ exhibits high CO₂ conversion and CO production even at a low temperature range. On the other hand, at temperatures 400°C or higher, CO accounted for 80% or more of the gas species produced in any of the samples. For Pt•NPs/Al₂O₃, the reaction of scheme (3) is considered to occur at 300°C, while the reaction of scheme (4) is considered to prevail at 400°C or higher.

CO₂ + 4H₂ → CH₄ + 2H₂O (3)

CO₂ + H₂ → CO + H₂O (4)

In addition, as the temperature increased, the reaction of scheme (1) with the generated CO and H₂ proceeded to yield CH₄. The resulting product selectivity thereby seemed to be decreased. However, the CO selectivity of PtW•NPs/Al₂O₃ was above 98% at 300°C, and at 400°C, the CO selectivity of 80% was also recorded. Further, Pt•NPs/Al₂O₃ also showed a similar trend in CO selectivity, but the CO₂ conversion was much lower than that of PtW•NPs/Al₂O₃ as described above. In (Pt•NPs+WO₃₋ₓ) /Al₂O₃, high CO selectivity was observed at 300-450°C, but the CO₂ conversion was much lower than in PtW•NPs/Al₂O₃ as shown above. PtW•NPs/Al₂O₃ has high CO₂ conversion and CO selectivity in any range at 300-450°C.

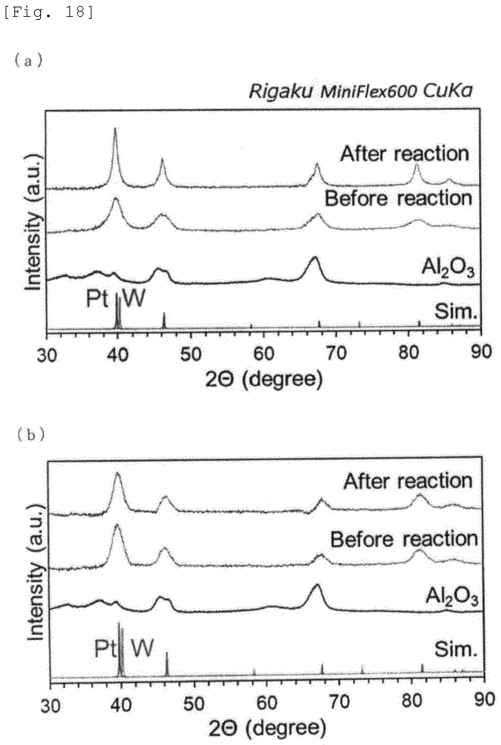

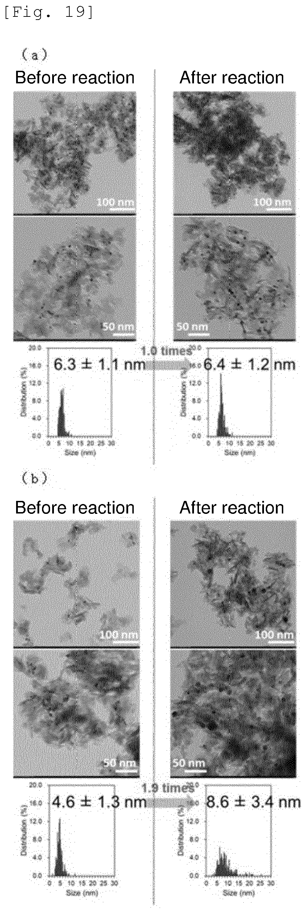

PtW•NPs/Al₂O₃ or Pt•NPs/Al₂O₃ before and after the CO₂ hydrogenation reaction was subjected to XRD measurement. Figure 18 shows the results. Figure 18(a) shows the results of PtW•NPs/Al₂O₃, and Figure 18(b) shows the results of Pt•NPs/Al₂O₃. Note that the baseline for alumina-carrier sample was subtracted, and fitting was performed by Le Bail fitting. The results of XRD measurement has demonstrated that the X-ray diffraction peaks of any of the samples were sharpened before and after the CO₂ hydrogenation reaction. The crystallite size was actually estimated from the fitting and compared between before and after the reaction. In the case of Pt•NPs/Al₂O₃, the size increase was 2.1-fold since the size was 6.0 nm before the reaction and 12.5 nm after the reaction. By contrast, in the case of PtW•NPs/Al₂O₃, the size increase was 1.2-fold since the size was 7.8 nm before the reaction and 9.1 nm after the reaction. In the case of PtW•NPs/Al₂O₃, an increase in the crystallite size was found to be suppressed. Further, a change in the particle size of PtW•NPs/Al₂O₃ or Pt•NPs/Al₂O₃ between before and after the CO₂ hydrogenation reaction was observed by TEM. Figure 19 shows the results. Figure 19(a) shows the results of PtW•NPs/Al₂O₃, and Figure 19(b) shows the results of Pt•NPs/Al₂O₃. The particle size was determined by TEM. In the case of Pt•NPs/Al₂O₃, a 1.9-fold increase in size was observed. In contrast, no size increase was observed for PtW•NPs/Al₂O₃. This has demonstrated that in the case of PtW•NPs/Al₂O₃, an increase in the particle size due to sintering can be suppressed even in the CO₂ hydrogenation reaction.

### [Example 6]

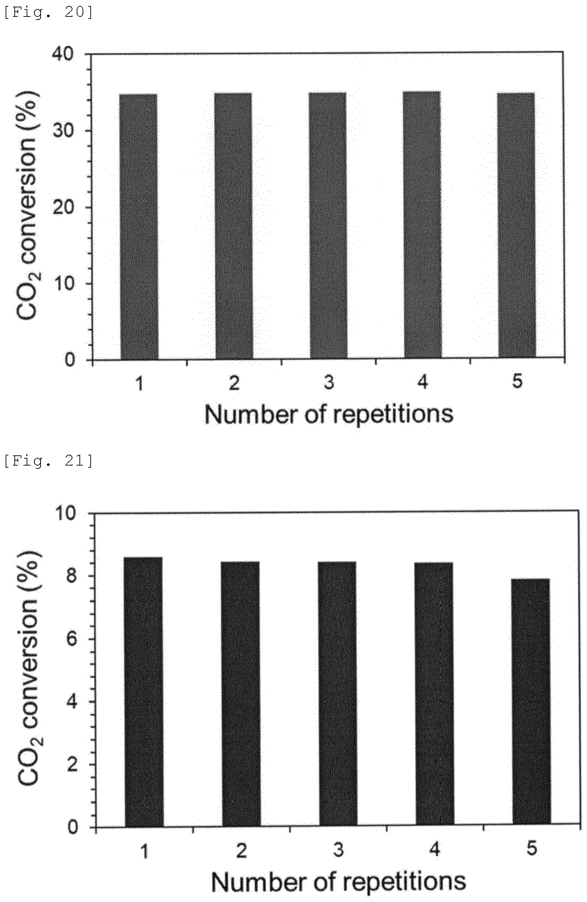

The same conditions and processes as in Example 5 at a temperature of 400°C were used to repeatedly conduct a durability test using PtW•NPs/Al₂O₃ (with 3.65 mg of Pt) sample. Figure 20 shows the results of measuring each CO₂ conversion during the five repetition tests. The CO₂ conversion of PtW•NPs/Al₂O₃ as measured after the fifth test remained the same. No decrease in the CO₂ conversion was observed. This indicates that PtW•NPs/Al₂O₃ has high durability.

### [Comparative Example 9]

A test was conducted in the same manner as in Example 6 except that PtW•NPs/Al₂O₃ was replaced by Pt•NPs/Al₂O₃. Figure 21 shows the results. The CO₂ conversion of Pt•NPs/Al₂O₃ from the initial time as measured after the fifth test was found to be decreased.

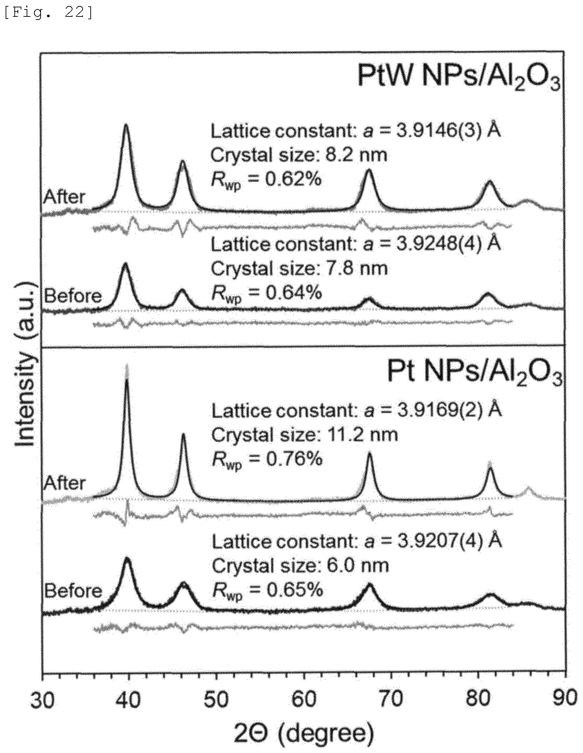

Figure 22 shows the powder X-ray diffraction patterns and crystallite sizes of PtW•NPs/Al₂O₃ or Pt•NPs/Al₂O₃ before the start of the test and after the fifth repetition test during the five repetition tests in Example 6 or Comparative Example 9. In the case of Pt•NPs/Al₂O₃, a 1.9-fold increase in crystallite size from the initial time was observed. On the other hand, little increase in crystallite size was observed in the case of PtW•NPs/Al₂O₃. In other words, PtW•NPs/Al₂O₃ has been demonstrated to have high durability in addition to the sintering suppression effect. Note that the same method as in the method shown in Figure 15 was adopted for the crystallite size fitting in Example 4 and Comparative Example 5 to obtain the results.

### [Example 7]

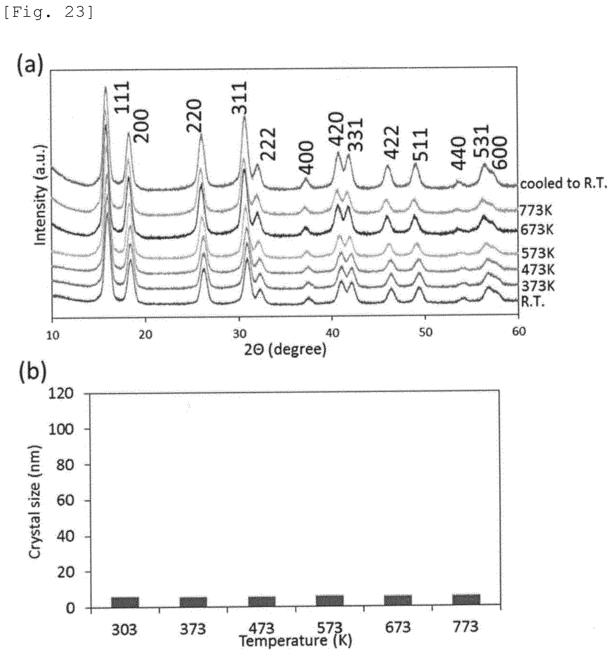

In situ-temperature-variable powder X-ray diffractometry of carrier-free PtW•NPs was performed under a hydrogen atmosphere (1-bar) while using synchrotron radiation at synchrotron radiation facility (SPring-8). Figure 23 shows the powder X-ray diffraction pattern and the crystallite size at each temperature.

### [Comparative Example 10]

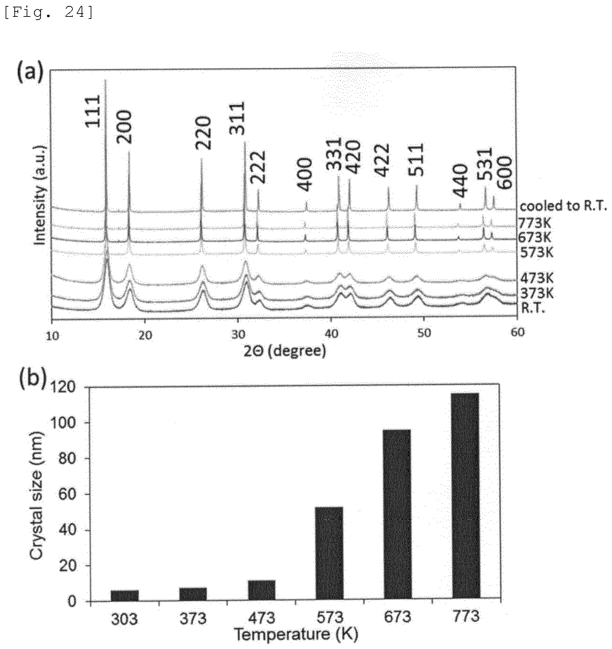

In situ-temperature-variable powder X-ray diffractometry of carrier-free Pt•NPs was performed under a hydrogen atmosphere while using synchrotron radiation at synchrotron radiation facility (SPring-8). Figure 24 shows the powder X-ray diffraction pattern and the crystallite size at each temperature.

Figure 23 shows that in the temperature range up to 773 K under hydrogen, PtW•NPs exhibited no change in the shape of the X-ray diffraction pattern and no increase in the crystallite size. By contrast, in the case of Pt•NPs, the peak shapes of the X-ray diffraction patterns became sharper as the temperature increased, and a remarkable increase in crystallite size was observed. The results have revealed that in the case of PtW•NPs, the effect of sintering in a reducing atmosphere can be suppressed. In addition, PtW•NPs in Example 7 is free of a carrier. Thus, it is suggested that the sintering suppression effect is not due to the effect of the carrier but to the effect of the solid solution of platinum and tungsten.

This has demonstrated that platinum-tungsten solid solution nanoparticles (PtW•NPs) elicit the sintering suppression effect even in hydrogen-involving catalytic reactions.

### Industrial Applicability

The platinum-tungsten solid solution particles or the catalyst containing the particles according to the present invention may be suitably utilized as a catalyst for various reactions, or other reactions, such as hydrogen evolution, carbon monoxide hydrogenation, carbon dioxide hydrogenation, acetylene hydrogenation, ethylene hydrogenation, formation of CO₂ from methane, aqueous gas shift, CO oxidation, and/or dehydrogenation (e.g., synthesis of benzene from cyclohexane).

## Claims

1. Platinum-tungsten solid solution particles comprising platinum and tungsten in solid solution at an atomic level.

2. A catalyst comprising the platinum-tungsten solid solution particles according to claim 1.

3. The catalyst according to claim 2, which is a hydrogen evolution catalyst.

4. The catalyst according to claim 2, which is a carbon monoxide hydrogenation catalyst.

5. The catalyst according to claim 2, which is a carbon dioxide hydrogenation catalyst.
